(19) **Europäisches Patentamt**
**European Patent Office**
**Office européen des brevets**

(11) **EP 3 205 622 B1**

(12) **EUROPÄISCHE PATENTSCHRIFT**

(45) Veröffentlichungstag und Bekanntmachung des Hinweises auf die Patenterteilung:
**09.05.2018 Patentblatt 2018/19**

(51) Int Cl.:
**C01B 3/34** (2006.01)   **C07C 29/151** (2006.01)
**C07C 31/04** (2006.01)

(21) Anmeldenummer: **16155353.2**

(22) Anmeldetag: **11.02.2016**

(54) **VERFAHREN ZUR SYNTHESE VON METHANOL**

METHOD FOR SYNTHESIS OF METHANOL

PROCEDE DE SYNTHESE DE METHANOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**16.08.2017 Patentblatt 2017/33**

(73) Patentinhaber:
- **Wagner, Ulrich**
  **06406 Bernburg OT Biendorf (DE)**
- **Balthasar, Wolff**
  **40833 Ratingen (DE)**
- **Müller, Dierk**
  **61184 Karben (DE)**

(72) Erfinder:
- **Wagner, Ulrich**
  **06406 Bernburg OT Biendorf (DE)**
- **Balthasar, Wolff**
  **40833 Ratingen (DE)**
- **Müller, Dierk**
  **61184 Karben (DE)**

(74) Vertreter: **Patentanwälte Bauer Vorberg Kayser Partnerschaft mbB**
**Goltsteinstraße 87**
**50968 Köln (DE)**

(56) Entgegenhaltungen:
**EP-A1- 2 281 793     WO-A1-96/21634**
**WO-A1-2006/126017**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die Erfindung betrifft ein Verfahren zur Synthese von Methanol gemäß dem Oberbegriff von Anspruch 1 sowie eine Anlage zur Synthese von Methanol gemäß dem Oberbegriff von Anspruch 15.

Methanol wird regelmäßig in großen Anlagen hergestellt, in denen fossile Energieträger wie etwa Kohle oder Erdgas in einem Zwischenschritt zunächst in ein Synthesegas aus Wasserstoff und Kohlenstoffoxiden wie insbesondere Kohlenmonoxid umgewandelt werden. Anschließend findet eine katalytische Umwandlung des Synthesegases in Methanol in einem entsprechenden Reaktor statt. Der methanolhaltige Produktstrom aus dem Reaktor wird dann abgekühlt und einem Abscheider zum Erhalten des Rohmethanols zugeführt, wobei ein verbliebenes Restgas ganz oder teilweise in einem Kreislauf durch den Reaktor gefahren wird.

Dabei kann jeweils sowohl Kohlenstoffmonoxid als auch Kohlenstoffdioxid mit Wasserstoff in an sich bekannter Weise katalytisch in Methanol und ggf. Wasser umgewandelt werden, wobei für eine möglichst vollständige Umsetzung des Kohlenstoffoxide und des Wasserstoffs ein molares Verhältnis S, welches durch

$$S = \frac{n(H_2) - n(CO_2)}{n(CO) + n(CO_2)}$$ mit n in [mol] gegeben ist, von im Wesentlichen 2,1 angestrebt wird. Dieses molare Verhältnis S wird hier und nachfolgend auch als Stöchiometriezahl bezeichnet.

Die aus dem Stand der Technik bekannte WO 96/21634 beschreibt ein Verfahren zur Herstellung von Methanol aus einem Synthesegas, welches aus einem kohlenstoffhaltigen Einsatzmaterial erhalten wurde, bei welchem Verfahren ein Teil des unreagierten Gasstroms aus einer ersten Methanolsynthesezone in die erste Methanolsynthesezone zurückgeführt wird, ein anderer Teil des unreagierten Gasstroms aus der ersten Methanolsynthesezone einer zweiten Methanolsynthesezone zugeführt wird, ein Teil des unreagierten Gasstroms aus der zweiten Methanolsynthesezone in die zweite Methanolsynthesezone zurückgeführt wird, Wasserstoff aus einem weiteren Teil des unreagierten Gasstroms aus der zweiten Methanolsynthesezone zurückgewonnen wird, um jeweils einen wasserstoffangereicherten und einen wasserstoffverarmten Gasstrom zu erhalten und der wasserstoffangereicherte Gasstrom in die zweite Methanolsynthesezone zurückgeführt wird.

Regelmäßig weist das Synthesegas aber eben nicht ein solches molares Verhältnis von im Wesentlichen 2,1 auf. Insbesondere bei der Gewinnung von Synthesegas mittels autothermer Reformierung liegt die Stöchiometriezahl etwa zwischen 1,6 und 1,8, sodass im Ergebnis der Anteil an Wasserstoff zu niedrig ist und bei der Methanolsynthese ein hoher Anteil an Kohlenstoffoxiden verbleibt. Da regelmäßig das verbleibende Gas im Kreislauf gefahren wird, führt die somit resultierende Erhöhung des zu rezyklierenden Gasvolumens zu einer drastischen Leistungsanforderung an die verwendeten Kompressoren und zu dem Erfordernis einer großen Katalysatormenge für die Methanolsynthese.

Aus dem Stand der Technik sind verschiedene Ansätze dafür bekannt, durch der Methanolsynthese prozesstechnisch vorgelagerter Zuspeisung von Wasserstoff die Stöchiometriezahl dem gewünschten Wert anzunähern. Die WO 2006/126017 A1, von welcher die vorliegende Erfindung ausgeht, schlägt zu diesem Zweck vor, einen Teil des nach der Methanolsynthese verbleibenden Gases im Anschluss an die Abtrennung eines Rohmethanols durch Kondensation als Purgegas abzuzweigen und etwa durch eine Druckwechsel-Adsorptionsvorrichtung, welche hier und nachfolgend auch als PSA (Pressure Swing Adsorption) bezeichnet wird, zu führen. Ebenso wird ein Teil des Synthesegases vor Eintritt in den Synthesekreislauf der PSA zugeführt. Der damit gewonnene Wasserstoff wird dem Synthesegasstrom zugeführt ebenso wie das nach der Methanolsynthese verbliebene und nicht abgezweigte Gas. Indem der Anteil des abgezweigten Gases variiert wird, kann die Stöchiometriezahl auf den gewünschten Wert eingestellt werden.

Nachteilig an diesem Ansatz ist allerdings, dass proportional zum zusätzlichen Gewinn von Wasserstoff für die Methanolsynthese die Kohlenstoffoxide in dem der PSA zugeführten Gas für die Methanolsynthese verloren gehen. Insofern reduziert sich die Ausbeute an Methanol bezogen auf die für die Synthesegaserzeugung eingesetzte Quelle. Ebenso ist es nachteilig, dass auch bei hinsichtlich der Stöchiometrie günstigeren Reaktionsbedingungen regelmäßig bei den hier anzusetzenden Molanteilen der Kohlenstoffoxide die Methanolsynthese aus dem Kohlenstoffmonoxid vorrangig vor der Methanolsynthese aus dem Kohlenstoffdioxid erfolgt. Die Folge ist, dass das Kohlenstoffdioxid aus dem Synthesegas nur unzureichend für die Methanolsynthese verwendet wird, deshalb auch weit gehend im Kreislauf gefahren wird ohne dass es zu einer Synthesereaktion kommt und es im Ergebnis nach dem Kreislauf zu großen Teilen durch die PSA abgeführt wird. Der insbesondere durch das mitgeführte Kohlenstoffdioxid erhöhte Volumenstrom im Kreislauf führt auch zu einem erhöhten Bedarf an Katalysatorvolumen.

Ausgehend von diesem Stand der Technik besteht die Aufgabe der Erfindung darin, das aus dem Stand der Technik bekannte Verfahren zur Methanolsynthese so weiterzuentwickeln und zu verbessern, dass eine gewünschte Stöchiometriezahl auch durch einen geringeren Verlust an Kohlenstoffoxiden erreichbar wird und auch die Wirtschaftlichkeit hinsichtlich der benötigten Kompressorleistung und der Katalysatormenge verbessert wird.

Bezogen auf ein Verfahren zur Synthese von Methanol gemäß dem Oberbegriff von Anspruch 1 wird diese Aufgabe durch den kennzeichnenden Teil des Anspruchs 1 gelöst. Bezogen auf eine Anlage zur Synthese von Methanol gemäß dem Oberbegriff von Anspruch 15 wird die Aufgabe durch den kennzeichnenden Teil des Anspruchs 15 gelöst.

Wesentlich für die Erfindung ist die Erkenntnis, dass dasjenige unreagierte Gas, welches nach der Methanolsynthese verbleibt und welches nicht einer Wasserstoffrückgewinnungsstufe wie speziell einer PSA zugeführt wird, einer der Hauptreaktorstufe nachgelagerten Nachreaktorstufe zur Methanolsynthese zugeführt werden kann. Auf diese Weise wird erreicht, dass auch das - nunmehr gegenüber dem Kohlenstoffmonoxid im Vergleich zu den Verhältnissen in der Hauptreaktorstufe - in der Nachreaktorstufe anteilig höher vorliegende Kohlenstoffdioxid zu großen Teilen in Methanol umgewandelt wird. Neben dieser unmittelbaren Verwertung des Kohlenstoffdioxids, welches ansonsten als Abgas abgegeben worden wäre, verringert sich auch die Menge des im Kreislauf gefahrenen Kohlenstoffdioxids und damit des Gasvolumens insgesamt. Dies entlastet die für den Kreislauf benötigten Kompressoren und erlaubt es, ein geringeres Katalysatorvolumen in den Reaktorstufen vorzusehen.

Die bevorzugten Ausgestaltungen der Unteransprüche 2 und 3 betreffen dabei spezielle molare Verhältnisse in den Gasströmen oder in den Reaktorstufen, welche für eine besonders umfassende Methanolsynthese durch beide Reaktorstufen insgesamt geeignet sind.

Die von dem Unteranspruch 4 vorgesehene Einstellbarkeit der Aufteilung der Gasströme für die Wasserstoffrückgewinnungsstufe einerseits und die Nachreaktorstufe andererseits erlaubt es, dynamisch auf veränderte Reaktionsbedingungen oder Zusammensetzungen des Synthesegasstroms zu reagieren.

Die Unteransprüche 7 und 8 wiederum beschreiben bevorzugte Ausgestaltungen zur Kühlung des Gasgemisches aus der Hauptreaktorstufe zwecks Abscheidung des Rohmethanols.

Der Unteranspruch 9 beschreibt eine besonders vorteilhafte Nutzung des Synthesegasstroms zur Kühlung der Nachreaktorstufe.

Der Unteranspruch 10 betrifft das vorteilhafte Vorsehen einer weiteren Trennstufe nach der Nachreaktorstufe und ein Rezyklieren des dabei verbleibenden unreagierten Gases als Nebenrecyclestrom, wobei der Unteranspruch 11 sich auf den vorteilhaft erhöhten Molanteil von Kohlenstoffdioxid darin bezieht.

Gemäß dem Unteranspruch 12 ist das vorgeschlagene Verfahren besonders für die Methanolsynthese aus Erdgas - und insbesondere Erdgas aus einer Vielzahl von Erdgasvorkommen - als Energieträger geeignet, da es die Abstimmung auf variierende Anteile unterschiedlicher Kohlenwasserstoffanteile in dem Erdgas erlaubt.

Der Unteranspruch 13 betrifft eine besonders vorteilhafte Methode zum Gewinnen des Synthesegases aus dem kohlenstoffhaltigen Energieträgerstrom durch autothermes Reformieren.

Der Unteranspruch 14 schließlich beschreibt eine weitere Ausgestaltung, mit der die Stöchiometriezahl für die Methanolsynthese auch bei variierenden Zusammensetzungen des kohlenstoffhaltigen Energieträgerstroms und/oder bei einem sehr hohen Gehalt an höheren Kohlenwasserstoffen durch einen zusätzlichen Ansatz eingestellt werden kann.

Weitere Einzelheiten, Merkmale, Ziele und Vorteile der vorliegenden Erfindung werden nachfolgend anhand der Zeichnung eines bevorzugten Ausführungsbeispiels erläutert. In der Zeichnung zeigt

Fig. 1    zeigt das Fließbild einer Anlage zur Ausführung des vorschlagsgemäßen Verfahrens gemäß einem ersten Ausführungsbeispiel,

Fig. 2    zeigt das Fließbild einer Anlage zur Ausführung des vorschlagsgemäßen Verfahrens gemäß einem zweiten Ausführungsbeispiel,

Fig. 3    zeigt das Fließbild einer ersten Variante einer Speiseanordnung für eine Anlage zur Ausführung des vorschlagsgemäßen Verfahrens und

Fig. 4    zeigt das Fließbild einer zweiten Variante einer Speiseanordnung für eine Anlage zur Ausführung des vorschlagsgemäßen Verfahrens.

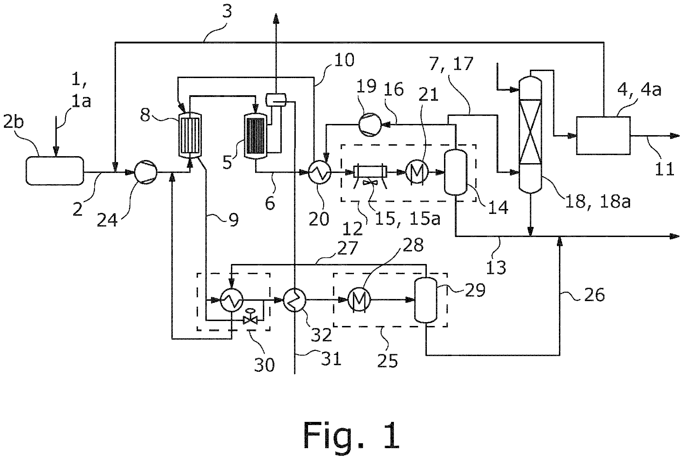

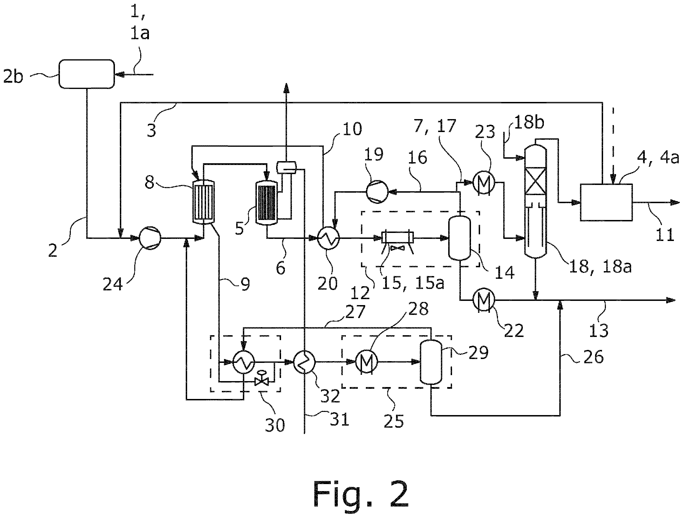

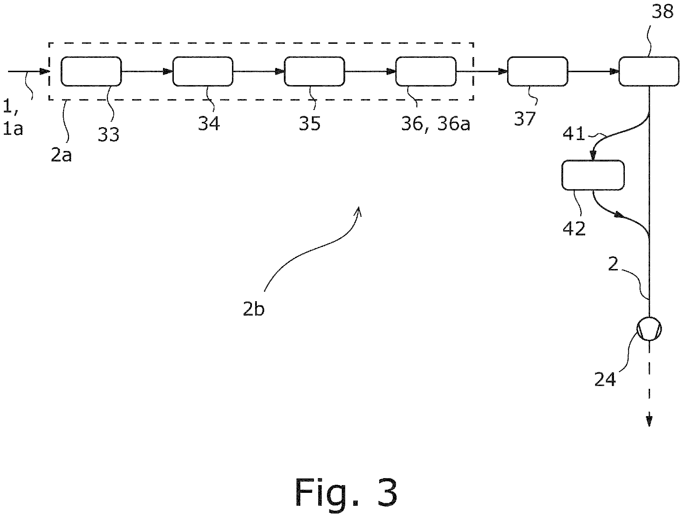

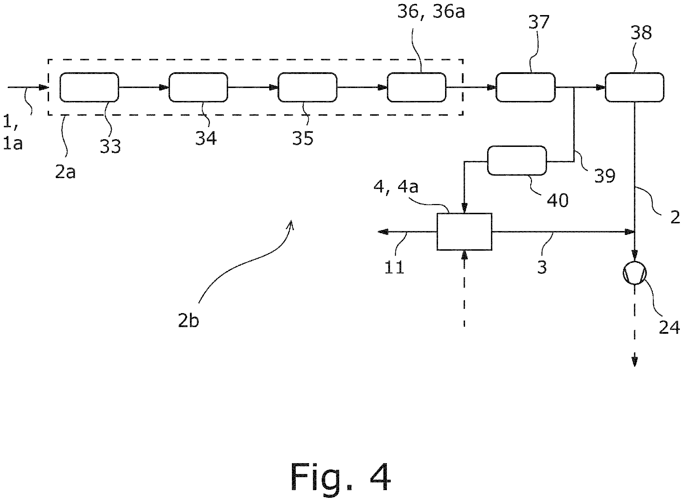

Das vorschlagsgemäße Verfahren dient der Synthese von Methanol und wird zunächst mit Bezug auf die Ausführungsbeispiele der Fig. 1 und Fig. 2 erläutert. Soweit nicht anders angegeben, beziehen sich die nachfolgenden Feststellungen gleichermaßen auf beide Ausführungsbeispiele. Bei dem vorschlagsgemäßen Verfahren wird einem Synthesegasstrom 2, welcher Wasserstoff und Kohlenstoffoxide umfasst, ein wasserstoffhaltiger Strom 3 aus einer Wasserstoffrückgewinnungsstufe 4 zugeführt und der Synthesegasstrom 2 wird einer Hauptreaktorstufe 5 zugeführt. Der Synthesegasstrom 2 kann prinzipiell aus beliebiger Quelle stammen, wird jedoch bevorzugt aus einem kohlenstoffhaltigen Energieträgerstrom 1 gewonnen, wobei wiederum die Art und Weise dieses Gewinnen des Synthesegasstroms 2 grundsätzlich beliebig sein kann. Auf die Einzelheiten bezüglich des Energieträgerstroms 1 und der Gewinnung des Synthesegasstroms 2 aus ihm bei den gegenständlichen Ausführungsbeispielen wird untenstehend und insbesondere in Bezug auf die Fig. 3 und 4 noch näher eingegangen. In dem Ausführungsbeispiel der Fig. 1 weist der gewonnene Synthesegasstrom 2 - also vor der untenstehend noch zu beschreibenden Zuführung von Recycleströmen o. dgl. - einen Volumenstrom von 601473 Nm$^3$/h auf und in dem Ausführungsbeispiel der Fig. 2 weist der gewonnene Synthesegasstrom 2 einen Volumenstrom von 602188 Nm$^3$/h auf.

Vorschlagsgemäß dient die Zuführung des Synthesegasstroms 2 zur Hauptreaktorstufe 5 der katalytischen und teilweisen Umsetzung des Synthesegasstrom 2 in ein Gasgemisch 6, welches Gasgemisch 6 Wasser, Methanol und Restgas umfasst. Diese Umsetzung erfolgt insoweit teilweise, dass unreagiertes Synthesegas als Bestandteil des Restgases verbleibt. Diese Hauptreaktorstufe 5 kann auch aus einer Vielzahl von prozesstechnisch parallel oder seriell angeordneten Einzelreaktoren bestehen, welche dann insgesamt die Hauptreaktorstufe 5 bilden.

Beispielhaft für die Ausführungsbeispiele der Fig. 1 und 2 gelangt der Synthesegasstrom 2 mit einem Druck von 74 bar und mit einer Temperatur von 210°C in die Hauptreaktorstufe 5. Im Ausführungsbeispiel der Fig. 1 weist dabei der zugeführte Synthesegasstrom 2 einen Volumenstrom von 1230677 Nm$^3$/h und im Ausführungsbeispiel der Fig. 2 einen Volumenstrom von 1201410 Nm$^3$/h auf. Die Erhöhung gegenüber den oben genannten Werten ergibt sich aus der noch zu beschreibenden Zuführung. Die exothermen Synthesereaktionen zu Methanol - welche an sich aus dem Stand der Technik bekannt sind - bewirken, dass das Gasgemisch 6 mit einer Temperatur von 256°C und mit einem Druck von 72,7 bar aus der Hauptreaktorstufe 5 tritt. In dem Ausführungsbeispiel der Fig. 1 weist das Gasgemisch 6 einen Volumenstrom von 1014516 Nm$^3$/h und in dem Ausführungsbeispiel der Fig. 2 einen Volumenstrom von 986507 Nm$^3$/h auf. Neben dem Wasserstoff und den Kohlenstoffoxiden des Synthesegases kann das Restgas weitere Bestandteile aufweisen, wobei hier insbesondere Inertgase infrage kommen. Als Katalysator für die in der Hauptreaktorstufe 5 erfolgenden und an sich bekannten Reaktionen für die Methanolsynthese kann beispielsweise ein Kupfer-Zinkoxid-Katalysator auf einem Aluminiumoxid-Träger verwendet werden.

Vorschlagsgemäß wird nun ein erster Teil 7 des Restgases der Wasserstoffrückgewinnungsstufe 4 zugeführt zur Trennung in den wasserstoffhaltigen Strom 3 und in einen Abgasstrom 11. Neben dem ersten Teil 7 des Restgases im engeren Sinne kann auch hier Methanol und Wasser mit der Wasserstoffrückgewinnungsstufe 4 zugeführt werden. Der Abgasstrom 11 kann dann verfeuert oder auf beliebige andere Weise verwendet werden. Bei der Wasserstoffrückgewinnungsstufe 4 handelt es sich hier und bevorzugt um eine Druckwechsel-Adsorptionsvorrichtung 4a (PSA). In diesem Fall besteht der wasserstoffhaltige Strom 3 im Wesentlichen aus Wasserstoff und der Abgasstrom 11 umfasst die nach Abtrennung des Wasserstoffs verbleibende Restmenge des ersten Teils 7 des Restgases. Prinzipiell kann aber auch ein anderer Ansatz zur Wasserstoffrückgewinnung durch Abtrennung des wasserstoffhaltigen Stroms 3 verwendet werden. Folglich kann der wasserstoffhaltige Strom 3 neben Wasserstoff auch weitere Stoffe umfassen. So kann die Wasserstoffrückgewinnungsstufe 4 eine Waschvorrichtung zur selektiven Ausschleusung von Kohlenstoffdioxid aufweisen oder sein, sodass der Abgasstrom 11 im Wesentlichen aus Kohlenstoffdioxid besteht. Entsprechend weist der wasserstoffhaltige Strom 3 dann neben Wasserstoff auch Inertgase auf. Bei dieser Variante kann vorzugsweise eine separate Abzweigung von Purgegas zur Abtrennung von Inertgasen vorgesehen sein, für deren prozesstechnische Platzierung grundsätzlich verschiedene Möglichkeiten bestehen. Im Ausführungsbeispiel der Fig. 1 wird der Wasserstoffrückgewinnungsstufe 4 ein Volumenstrom von 240182 Nm$^3$/h und im Ausführungsbeispiel der Fig. 2 ein Volumenstrom von 220708 Nm$^3$/h zugeführt. Der wasserstoffhaltige Strom 3 im Ausführungsbeispiel der Fig. 1 weist einen Volumenstrom von 160312 Nm$^3$/h und im Ausführungsbeispiel der Fig. 2 einen Volumenstrom von 142570 Nm$^3$/h auf.

Das vorschlagsgemäße Verfahren ist nun dadurch gekennzeichnet, dass ein zweiter Teil 10 des Restgases einer Nachreaktorstufe 8 zur weiteren katalytischen und zumindest teilweisen Umsetzung in einen methanolhaltigen Produktstrom 9 zugeführt wird. In der Nachreaktorstufe 8 laufen die im Grunde gleichen Reaktionen zur Synthese von Methanol wie in der Hauptreaktorstufe 5 ab, wobei die Nachreaktorstufe 8 bzw. die in ihr ablaufende Synthesereaktion jedoch hinsichtlich der Art und Auslegung des Reaktors, hinsichtlich des verwendeten Katalysators und hinsichtlich der Randbedingungen der Synthesereaktion - insbesondere Menge und Anteil der Edukte, Druck, Temperatur - im Prinzip beliebig und regelmäßig verschieden zur Hauptreaktorstufe 5 bzw. zur Synthesereaktion in der Hauptreaktorstufe 5 sein wird. Während in der Hauptreaktorstufe 5 regelmäßig 80 % des vorhandenen Kohlenstoffmonoxids zu Methanol umgewandelt wird, erfolgt eine Umwandlung zu Methanol des Kohlenstoffdioxids lediglich zu etwa 30 % bis 40 %. Durch den deutlich höheren Molanteil von Kohlenstoffdioxid gegenüber Kohlenstoffmonoxid in der Nachreaktorstufe 8 überwiegt hier wiederum die Umwandlung von Kohlenstoffdioxid zu Methanol. Dies wird untenstehend noch genauer beschrieben.

So wie oben bereits für die Hauptreaktorstufe 5 festgestellt, kann auch die Nachreaktorstufe 8 aus einer Vielzahl von prozesstechnisch parallel oder seriell angeordneten Einzelreaktoren bestehen. Diese bilden dann insgesamt die Nachreaktorstufe 8. Vorliegend erfolgt ein Eintritt von Gas in die Nachreaktorstufe 8 - welches den zweiten Teil 10 des Restgases umfasst - mit einer Temperatur von 210° C und mit einem Druck von 82,6 bar, basierend auf einem weiter unten beschriebenen Kompressor. Neben dem zweiten Teil 10 des Restgases 6 im engeren Sinne kann auch Methanol und Wasser mit in die Nachreaktorstufe 8 treten. Der durch die Methanolsynthese in der Nachreaktorstufe 8 erhaltene methanolhaltige Produktstrom 9 tritt hier beispielhaft mit einer Temperatur von 220°C und mit einem Druck von 77,8 bar aus. Auch in der Nachreaktorstufe 8 kann eine Umsetzung stattfinden, welche nur teilweise erfolgt, sodass auch der methanolhaltige Produktstrom 9 regelmäßig Methanol, Wasser und Restgas umfasst. Dabei kann auch hier unreagiertes Synthesegas verbleibender Bestandteil des Restgases sein.

Eine bevorzugte Ausführungsform ist dadurch gekennzeichnet, dass der Synthesegasstrom 2 vor Zuführung des wasserstoffhaltigen Stroms 3 ein molares Verhältnis von S < 2 aufweist. Dieses molare Verhältnis S ist gegeben durch $S = \frac{n(H_2) - n(CO_2)}{n(CO) + n(CO_2)}$ mit n in [mol]. Damit weist der Synthesegasstrom 2 also für die Methanolsynthese einen zu geringen Anteil an Wasserstoff auf. Diese bevorzugte Ausführungsform ist ferner dadurch gekennzeichnet, dass in der Hauptreaktorstufe 5 ein molares Verhältnis von S > 2, vorzugsweise von S > 3 und insbesondere von im Wesentlichen S = 4 vorliegt. Mit dem molaren Verhältnis in der Hauptreaktorstufe 5 ist das molare Verhältnis S gemeint, welches sich aus den molaren Anteilen von Wasserstoff, Kohlenstoffmonoxid und Kohlenstoffdioxid in der Hauptreaktorstufe 5 gemäß der genannten Formel ergibt. Auch durch die obige Zuführung des wasserstoffhaltigen Stroms 3 wird also sogar ein Überschuss an Wasserstoff in der Hauptreaktorstufe 5 erreicht, was sich vorteilhaft auf die Reaktionsgeschwindigkeit und die Produktqualität auswirkt, sodass die Entstehung von Nebenprodukten eingeschränkt wird.

Eine weitere bevorzugte Ausführungsform ist dadurch gekennzeichnet, dass in der Nachreaktorstufe 8 ein molares Verhältnis S vorliegt - wiederum gegeben durch $S = \frac{n(H_2) - n(CO_2)}{n(CO) + n(CO_2)}$ mit n in [mol] - welches größer ist als das in der Hauptreak-torstufe 5 vorliegende molare Verhältnis S. Vorzugsweise ist es mindestens um den Faktor 1,5 größer oder im Wesentlichen um den Faktor 1,5 größer. Sinngemäß gleich zu der Hauptreaktorstufe 5 ist mit dem molaren Verhältnis in der Nebenreaktorstufe 8 das molare Verhältnis S gemeint, welches sich aus den molaren Anteilen von Wasserstoff, Kohlenstoffmonoxid und Kohlenstoffdioxid in der Nebenreaktorstufe 8 gemäß der genannten Formel ergibt. Der molare Anteil von Kohlenstoffmonoxid an der Summe der Molmenge von Wasserstoff, Kohlenstoffmonoxid und Kohlenstoffdioxid in der Nachreaktorstufe 8 ist geringer als der molare Anteil von Kohlenstoffmonoxid an der Summe der Molmenge von Wasserstoff, Kohlenstoffmonoxid und Kohlenstoffdioxid in der Hauptreaktorstufe 5. Mit anderen Worten ist in Relation zu der gesamten Stoffmenge aus den genannten Stoffen der molare Anteil von Kohlenstoffmonoxid in der Nachreaktorstufe 8 geringer als der molare Anteil von Kohlenstoffmonoxid in der Hauptreaktorstufe 5. Damit wird erreicht, dass in der Nachreaktorstufe 8 verstärkt die Umwandlung von Kohlenstoffdioxid zu Methanol stattfindet, insbesondere weil nicht mehr ausreichend Kohlenstoffmonoxid für die diesbezügliche Synthese vorhanden ist.

Es ist auch eine bevorzugte Ausführungsform vorgesehen, gemäß der und wie in den Fig. 1 und 2 dargestellt der erste Teil 7 des Restgases und der zweite Teil 10 des Restgases durch eine Stromaufteilung des Gasgemisches 6 in mindestens einen Hauptrecyclestrom 16 zur Zuführung in die Nachreaktorstufe 8 und in einen Purgestrom 17 zur Zuführung zu der Wasserstoffrückgewinnungsstufe 4 getrennt werden. Dabei umfasst der Hauptrecyclestrom 16 den zweiten Teil 10 des Restgases und der Purgestrom 17 den ersten Teil 7 des Restgases. Hier ist weiter bevorzugt, dass das Volumenverhältnis des Hauptrecyclestroms 16 zu dem Purgestrom 17 einstellbar ist. Eine entsprechende Ventilanordnung 17a zur Einstellung dieses Volumenverhältnisses ist schematisch in den Fig. 1 und 2 wiedergegeben. Besonders vorteilhaft ist die Möglichkeit, dass das Volumenverhältnis des Hauptrecyclestroms 16 zu dem Purgestrom 17 basierend auf einer Zusammensetzung des Synthesegasstroms 2 eingestellt wird. Diese Zusammensetzung des Synthesegasstroms 2, und zwar insbesondere unmittelbar vor Zuführung zur Hauptreaktorstufe 5, kann durch eine hier ebenfalls schematisch dargestellte Messanordnung 17b erfasst werden. Auf diese Weise kann auf auch dynamische Veränderungen z. B. des molaren Wasserstoffanteils im Synthesegasstrom 2 durch Steigern oder Absenken der Stoffmenge des wasserstoffhaltigen Stroms 3 reagiert werden.

Um in dem Purgestrom 17 verbliebenes Methanol zu gewinnen, kann der Purgestrom 17 - wie in den Figuren dargestellt - vor Zuführung zu der Wasserstoffrückgewinnungsstufe 4 einer Waschstufe 18 zum Auswaschen von verbliebenem Methanol in dem Purgestrom 17 zugeführt werden. Auf diese Weise kann erreicht werden, dass im Wesentlichen das Restgas ohne Methanol der Wasserstoffrückgewinnungsstufe 4 zugeführt wird. Diese Waschstufe 18 besteht vorliegend aus einem Gaswäscher 18a. In dem ersten Ausführungsbeispiel der Fig. 1 wird ein Purgestrom 17 mit einem Volumenstrom von 240998 Nm$^3$/h bei 40°C und mit einem Druck von im Wesentlichen 70 bar dem Gaswäscher 18a zugeführt, in welchem die Wäsche mit 5000 kg/h Wasser bei 40°C erfolgt. Dieses Wasser wird durch eine Wasserzufuhr 18b bereitgestellt. Auf diese Weise wird ein Rohmethanol ausgewaschen, welches 1515 kg/h an Methanol enthält.

Gemäß den Ausführungsbeispielen kann dann der Hauptrecyclestrom 16 mit dem zweiten Teil 10 des Restgases der Nachreaktorstufe 8 zugeführt werden, wobei dabei bevorzugt wird, dass der Hauptrecyclestrom 16 zur Komprimierung durch einen Recyclekompressor 19 gefahren wird. Im Ausführungsbeispiel der Fig. 1 weist der Hauptrecyclestrom 16 einen Volumenstrom von 641255 Nm$^3$/h und im Ausführungsbeispiel der Fig. 2 einen Volumenstrom von 645472 Nm$^3$/h auf. In den vorliegenden Ausführungsbeispielen erhöht der Recyclekompressor 19 auf diese Weise den Druck des Hauptrecyclestroms 16 von hier im Wesentlichen 70 bar auf 83,1 bar. Ebenso kann der Hauptrecyclestrom 16 zur Kühlung des Gasgemischs 6 durch einen Hauptrecycle-Wärmetauscher 20 geführt werden. Wie aus den Fig. zu erkennen ist, wird zur Kühlung des Gasgemischs 6 auch das Gasgemisch 6 durch den Hauptrecycle-Wärmetauscher 20 geführt. Dies führt auch zu einer Erhitzung des Hauptrecyclestroms 16 auf die oben bereits erwähnte Temperatur von 210°C.

Bevorzugt und wie in den Ausführungsbeispielen dargestellt wird das Gasgemisch 6 gekühlt und speziell einer Trennstufe 12 zum Abtrennen eines Rohmethanolstroms 13 zugeführt. Bevorzugt und wie in den Ausführungsbeispielen dargestellt wird das Gasgemisch 6 im Wesentlichen vollständig der Trennstufe 12 zugeführt. Der Rohmethanolstrom 13 umfasst Wasser und Methanol und kann insbesondere im Wesentlichen aus Wasser und Methanol bestehen. Regelmäßig erfolgt kein Trennen des gesamten Methanols oder des gesamten Wassers aus dem Gasgemisch 6 durch diese Trennstufe 12, sondern nur jeweils eines Teils. Bevorzugt weist die Trennstufe 12 eine Abscheidestufe 14 auf, welche den Rohmethanolstrom 13 als Kondensat des Gasgemisches 6 abtrennt. In den Ausführungsbeispielen tritt das nach der Abscheidung verbliebene Gasgemisch 6 - also das Restgas zusammen mit dem unkondensierten Methanol und Wasser - mit einem Druck von im Wesentlichen 70 bar aus der Trennstufe 12 und insbesondere aus der Abscheidestufe 14 aus. Aus einem Sumpf des Gaswäschers 18a gelangt dieses Methanol in den Rohmethanolstrom 13 aus der Abscheidestufe 14, welcher im Ausführungsbeispiel der Fig. 1 151347 kg/h Methanol in dem Rohmethanol enthält.

Hier kann es weiter bevorzugt sein, dass die Trennstufe 12 eine der Abscheidestufe 14 vorgelagerte Kühlstufe 15 - bei welcher es sich insbesondere um eine Luftkühlstufe 15a handeln kann - zur Abkühlung des Gasgemisches 6 aufweist. Dabei sieht das zweite Ausführungsbeispiel der Fig. 2 beispielhaft eine Abkühlung durch diese Kühlstufe 15 auf eine Kühltemperatur oberhalb des Siedepunkts von Methanol bei Normaldruck vor und speziell eine Abkühlung auf eine Temperatur von 70°C vor.

Weiter ist es bevorzugt, dass die obige Kühlung des Gasgemischs 6 vor einer Aufteilung in den ersten Teil 7 und den zweiten Teil 10 des Restgases erfolgt. Je tiefer die Temperatur in der Abscheidestufe 14 ist, umso mehr Methanol kann aus dem Gasgemisch 6 abgetrennt werden. Daher ist einer bevorzugten Ausführungsform vorgesehen, welche hier in dem ersten Ausführungsbeispiel der Fig. 1 verwirklicht ist, dass die Trennstufe 12 eine der Kühlstufe 15 prozesstechnisch nachgelagerte weitere Kühlstufe 21 aufweist. Das bedeutet, dass das Gasgemisch 6 zunächst durch die Kühlstufe 15 und anschließend durch die weitere Kühlstufe 21 geführt wird. Bevorzugt ist weiter, dass die Kühlstufe 15 und die weitere Kühlstufe 21 das Gasgemisch 6 auf weniger als 45°C kühlen. Nach Durchlauf der beiden Kühlstufen 15, 21 beträgt also die Temperatur des Gasgemisches 6 weniger als 45°C. Im vorliegenden Ausführungsbeispiel der Fig. 2 wird das Gasgemisch 6 auf 40°C gekühlt, bei welcher Temperatur das Gasgemisch 6 in die Abscheidestufe 14 tritt.

Es ist bevorzugt, dass nach Abtrennen des Rohmethanolstroms 13 das verbliebene Gasgemisch 6 in den Hauptrecyclestrom 16 und in den Purgestrom 17 getrennt wird.

Bezüglich der weiteren Verarbeitung nach der Abscheidestufe 14 ist es bevorzugt, dass der Purgestrom 17 von der Abscheidestufe 14 der Waschstufe 18 - hier dem Gaswäscher 18a - bei im Wesentlichen gleichbleibender Temperatur zugeführt wird, in dem vorliegenden Beispiel also bei einer Temperatur von 40°C. Alternativ oder zusätzlich kann - wie ebenfalls dargestellt - vorgesehen sein, dass der Hauptrecyclestrom 16 von der Abscheidestufe 14 dem Recyclekompressor 19 bei im Wesentlichen gleichbleibender Temperatur zugeführt wird.

Statt für die Abscheidestufe 14 eine möglichst tiefe Temperatur zu erreichen, kann ein größerer Teil der Methanolabtrennung aber auch in die obige Waschstufe 18 verlagert werden. Hierfür sieht eine weitere bevorzugte Ausführungsform, welche in dem zweiten Ausführungsbeispiel der Fig. 2 verwirklicht ist, vor, dass der Rohmethanolstrom 13 einer Rohmethanolkühlstufe 22 zur Abkühlung des Rohmethanolstroms 13 zugeführt wird, wobei vorzugsweise eine Abkühlung auf unter 45°C erfolgt. Im Ausführungsbeispiel der Fig. 2 findet eine Abkühlung auf 40°C statt. Weiter kann der Purgestrom 17 vor Zuführung zu der Waschstufe 18 einer Purgestromkühlstufe 23 zur Abkühlung des Purgestroms 15 zugeführt werden, wobei hier ebenfalls bevorzugt eine Abkühlung auf unter 45°C erfolgt. Gemäß dem Ausführungsbeispiel der Fig. 2 findet eine Abkühlung speziell auf 40°C statt. Ohne die weitere Kühlstufe 21 des ersten Ausführungsbeispiels in der Fig. 1 kühlt lediglich der Luftkühler 15a vor der Abscheidestufe 14 in dem zweiten Ausführungsbeispiel der Fig. 2, sodass die Temperatur in der Abscheidestufe 14 70°C statt 40°C beträgt.

Im Ergebnis sieht also das zweite Ausführungsbeispiel der Fig. 2 zwei der Abscheidestufe 14 in jeweils unterschiedlichen Zweigen nachgelagerte Kühlstufen - nämlich die Rohmethanolkühlstufe 22 und die Purgestromkühlstufe 23 - statt einer zusätzlichen weiteren Kühlstufe 21 vor der Abscheidestufe 14 vor. Dies führt dazu, dass bei dem zweiten Ausführungsbeispiel durch die beschriebene höhere Temperatur in der Abscheidestufe 14 nur noch 136644 kg/h Methanol in dem Rohmethanolstrom 13 anfallen. Bei Zuführung des Purgestroms 17 mit einem Volumenstrom von 224100 Nm$^3$/h an die Waschstufe 18, ebenfalls bei 40°C durch die Purgestromkühlstufe 23, und dem Vorsehen von 2000 kg/h Wasser bei 40°C, wird nun hier ein Rohmethanol ausgewaschen, welches 4846 kg/h an Methanol enthält. Aus dem Sumpf des Gaswäschers 18a gelangt dieses Methanol wiederum in den Rohmethanolstrom 13 aus der Abscheidestufe 14, und zwar - wie in der Fig. 2 dargestellt - der obigen Rohmethanolkühlstufe 22 prozesstechnisch nachgelagert.

Der Gaswäscher 18a des zweiten Ausführungsbeispiels der Fig. 2 unterscheidet sich von dem Gaswäscher 18a der Fig. 1 darin, dass die Packung innerhalb der Kolonne weiter entfernt vom Grund der Kolonne endet, u.a. weil, wegen der erwartbar größeren Menge an ausgewaschenem Methanol, ein höherer Pegel des Sumpfes erwartet wird. Ferner weist der Gaswäscher 18a des zweiten Ausführungsbeispiels eine Abscheideranordnung zwischen dieser Packung und dem Grund der Kolonne auf. Ein weiterer Effekt der Unterschiede in der Kühlung ist, dass der Recyclekompressor 19 in dem ersten Ausführungsbeispiel der Fig. 1 mit geringerer Leistung gegenüber dem zweiten Ausführungsbeispiel der Fig. 2 betrieben werden kann.

Ebenso unterschiedlich für die Ausführungsbeispiele der Fig. 1 und 2 ist der Volumenstrom des wasserstoffhaltigen Stroms 3 aus der Druckwechsel-Adsorptionsvorrichtung 4. Im ersten Ausführungsbeispiel (Fig. 1) entspricht der Normvolumenstrom des wasserstoffhaltigen Stroms 3 148140 Nm3/h und im zweiten Ausführungsbeispiel (Fig. 2) 142570 Nm3/h.

Die Reaktion für die Methanolsynthese ist druckabhängig und wird durch höheren Druck befördert. Entsprechend wird gemäß einer bevorzugten Ausführungsform und wie dargestellt der Synthesegasstrom 2 zur Komprimierung durch einen Synthesegaskompressor 24 gefahren. In den vorliegenden Ausführungsbeispielen wird Synthesegas, welches bei 40°C und einem Druck von 56,3 bar zugeführt wird, durch den Synthesegaskompressor 24 auf 75 bar gebracht. Da der obige Recyclekompressor 19 nur eine geringere Menge als der Synthesegaskompressor 24 zu komprimieren braucht, kann er mit einem höheren Druck betrieben werden. Das ist einerseits für die Synthesereaktion in der Nachreaktorstufe 8 vorteilhaft, andererseits kann es von der Notwendigkeit entbinden, auch das nach der - unten beschriebenen - weiteren Trennstufe 25 verbliebene Gas wieder zu komprimieren. Hierzu ist es bevorzugt, dass der Enddruck des Recyclekompressors 19 höher ist als der Enddruck des Synthesegaskompressor 24 und insbesondere, dass der Enddruck des Recyclekompressors 19 um mindestens 10% höher ist als der Enddruck des Synthesegaskompressors 24.

Hier ist insbesondere vorgesehen, dass der Synthesegasstrom 2 vor Zuführung zu der Hauptreaktorstufe 5 der Nachreaktorstufe 8 zu ihrer Gaskühlung zugeführt wird. Wenn es sich also, wie in den vorliegenden Ausführungsbeispielen, bei der Nachreaktorstufe 8 um einen gasgekühlten Reaktor handelt, dann stellt gemäß dieser Variante der Synthesegasstrom 2 das Gas für die Kühlung der Nachreaktorstufe 8 bereit. Wie in den Figuren dargestellt, wird bevorzugt der wasserstoffhaltige Strom 3 dem Synthesegasstrom 2 prozesstechnisch dem Synthesegaskompressor 24 vorgelagert zugeführt wird. Aus der Wasserstoffrückgewinnungsstufe 4 tretend wird der wasserstoffhaltige Strom 3 regelmäßig keinen ausreichenden Druck haben.

Grundsätzlich kann es ausreichen, dass auch bei einer Anordnung mit zwei oder mehr Reaktoren nur eine einzelne Trennstufe zum Abtrennen von Methanol vorgesehen ist. Gemäß den dargestellten Ausführungsbeispielen ist es jedoch bevorzugt, dass der methanolhaltige Produktstrom 9 einer weiteren Trennstufe 25 zum Abtrennen eines weiteren Rohmethanolstrom 26, im Wesentlichen bestehend aus Wasser und Methanol, zugeführt wird. In dem ersten Ausführungsbeispiel der Fig. 1 enthält der weitere Rohmethanolstrom 26 74689 kg/h Methanol in dem Rohmethanol. Damit werden insgesamt - also durch diesen weiteren Rohmethanolstrom 26 sowie durch das jeweilige Rohmethanol aus der Abscheidestufe 14 und der Waschstufe 18 - 227551 kg/h Methanol gewonnen.

U.a. bedingt durch die insgesamt geringere Abtrennung von Rohmethanol durch die Trennstufe 12 und die Waschstufe 18 des zweiten Ausführungsbeispiels enthält der weitere Rohmethanolstrom 26 bei dem zweiten Ausführungsbeispiel der Fig. 2 88319 kg/h Methanol in dem Rohmethanol. In dem zweiten Ausführungsbeispiel werden folglich insgesamt 229859 kg/h Methanol gewonnen.

Auch nach diesem Abtrennen des weiteren Rohmethanolstroms 26 verbleibt ein Restgas. Entsprechend ist es bevorzugt, dass ein nach Abtrennen des weiteren Rohmethanolstroms 26 verbliebener Nebenrecyclestrom 27 - welcher hier das Restgas des methanolhaltigen Produktstroms 9 umfasst oder im Wesentlichen aus dem Restgas besteht - dem Synthesegasstrom 2 zugeführt wird. Da, wie oben bereits beschrieben, in den vorliegenden Ausführungsbeispielen der Recyclekompressor 19 mit einem Enddruck betrieben wird, welcher den Enddruck des Synthesegaskompressors 24 übersteigt, kann gemäß einer bevorzugten und hier dargestellten Ausführungsform der Nebenrecyclestrom 27 dem Synthesegasstrom 2 prozesstechnisch dem Synthesegaskompressor 24 nachgelagert zugeführt werden. Indem der Synthesegaskompressor 24 das entsprechende Volumen also nicht selbst verarbeiten muss, wird der Synthesegaskompressor 24 entlastet. In den vorliegenden Ausführungsbeispielen tritt der Nebenrecyclestrom 27 mit 75,9 bar aus der weiteren Trennstufe 25, was dem Enddruck des Synthesegaskompressors 24 von 75 bar hinreichend nahe kommt. Bei dem Ausführungsbeispiel der Fig. 1 weist der Nebenrecyclestrom 27 einen Volumenstrom von 468892 Nm$^3$/h und im Ausführungsbeispiel der Fig. 2 einen Volumenstrom von 456652 Nm$^3$/h auf.

Eine bevorzugte Ausführungsform sieht hier weiter vor, dass der Nebenrecyclestrom 27 einen Molanteil an Kohlenstoffdioxid aufweist, welcher mindestens doppelt so hoch wie der Molanteil des Nebenrecyclestroms 27 an Kohlenstoffmonoxid ist. Dies bedeutet, dass das Kohlenstoffmonoxid in der Nachreaktorstufe 8 weit gehend zu Methanol umgewandelt wurde, was wiederum die dann verstärkte Umwandlung des Kohlenstoffdioxids in Methanol ermöglicht. Auf diese Weise kann das Kohlenstoffdioxid wirksam für die Methanolsynthese eingesetzt werden.

In Analogie zur Trennstufe 12 nach der Hauptreaktorstufe 5 kann die weitere Trennstufe 25 eine Nach-Kühlstufe 28 und eine Nach-Abscheidestufe 29 umfassen, wobei der methanolhaltige Produktstrom 9 in den vorliegenden Ausführungsbeispielen auf 40°C gekühlt und mit einem Druck von 76 bar aus der Nach-Kühlstufe 28 tritt.

Entsprechend hat auch der Nebenrecyclestrom 27 im Wesentlichen diese niedrige Temperatur, welche vorteilhafterweise zur Vorkühlung des hier mit 220°C aus der Nachreaktorstufe 8 tretenden methanolhaltigen Produktstroms 9 verwendet werden kann. Daher ist es bevorzugt, dass der Nebenrecyclestrom 27, insbesondere vor Zuführung zum Synthesegasstrom 2, durch eine trimmbare Wärmetauscheranordnung 30 zur Kühlung des methanolhaltigen Produktstroms 9 geführt wird, indem auch der methanolhaltige Produktstrom 9 durch die Wärmetauscheranordnung 30 geführt wird. Die Kühlung des methanolhaltigen Produktstroms 9 erfolgt also dadurch, dass in der Wärmetauscheranordnung 30 ein Temperaturaustausch zwischen dem methanolhaltigen Produktstrom 9 und dem Nebenrecyclestrom 27 stattfindet. Die Trimmbarkeit der Wärmetauscheranordnung 30 kann prinzipiell beliebig und wie in den Ausführungsbeispielen dadurch erreicht werden, dass ein insbesondere variabler Teil des methanolhaltigen Produktstroms 9 um den Temperaturaustausch mit dem Nebenrecyclestrom 27 herumgeführt wird.

Gemäß einer bevorzugten Ausführungsform handelt es sich bei Hauptreaktorstufe 5 um einen isothermen und wassergekühlten Reaktor. Entsprechend kann es sein, dass der Hauptreaktorstufe 5 Speisewasser 31 zur Wasserkühlung zugeführt wird. Auch dessen vergleichsweise niedrige Ausgangstemperatur kann zu einer Vorkühlung des methanolhaltigen Produktstroms 9 verwendet werden, und zwar dadurch, dass das Speisewasser 31 vor Zuführung zur Hauptreaktorstufe 5 durch einen Wasserwärmetauscher 32 geführt wird, durch welchen auch der methanolhaltige Produktstrom 9 zum Wärmeaustausch geführt wird. In dem Wasserwärmetauscher 32 findet also zwecks Kühlung ein Temperaturaustausch zwischen dem Speisewasser 31 und dem methanolhaltigen Produktstrom 9 statt.

Wie bereits erwähnt, wird der Synthesegasstrom 2 bevorzugt aus dem obigen kohlenstoffhaltigen Energieträgerstrom 1 gewonnen. Dieser kohlenstoffhaltige Energieträgerstrom 1 wiederum kann grundsätzlich beliebiger Art sein. Das vorschlagsgemäße Verfahren hat sich dann als besonders geeignet erwiesen, wenn - wie bevorzugt - der kohlenstoffhaltige Energieträgerstrom 1 einen Erdgasstrom 1a umfasst oder im Wesentlichen aus diesem Erdgasstrom 1a besteht. Folglich kann der Energieträgerstrom 1 Methan, Ethan, Propan und teilweise auch Butan umfassen. Es kann auch sein, dass der Erdgasstrom 1a aus einer Vielzahl von geografisch verteilten Erdgasvorkommen gespeist wird. Hieraus kann sich eine besonders ungleichmäßige Verteilung der o.g. Bestandteile des Erdgases ergeben, was wiederum Auswirkungen auf die relativen Molanteile von Wasserstoff, Kohlenstoffmonoxid und Kohlenstoffdioxid hat. Das vorschlagsgemäße Verfahren erlaubt es, sich auf den ggf. mehr oder weniger stark ausgeprägten Mangel an Wasserstoff durch Einstellen der Aufteilung zwischen dem ersten Teil 7 und dem zweiten Teil 10 des Restgases einzurichten.

Ein erstes und ein zweites bevorzugtes Ausführungsbeispiel einer Speiseanordnung 2b zum Bereitstellen eines Synthesegasstroms 2 ist jeweils in der Fig. 3 bzw. in der Fig. 4 dargestellt. Dabei sind beide Varianten der Speiseanordnung 2b im Grunde beliebig mit den jeweiligen Ausführungsbeispielen der Fig. 1 und Fig. 2 kombinierbar. Die nachstehenden Ausführungen zu der Speiseanordnung 2b betreffen grundsätzlich, soweit nicht anders vermerkt, sowohl die Variante der Fig. 3 als auch die Variante der Fig. 4.

Das Gewinnen des Synthesegasstroms 2 aus dem Erdgasstrom 1a erfolgt bevorzugt in an sich bekannter Weise dadurch, dass der Erdgasstrom 1a zwecks Entschwefelung einer Entschwefelungsvorrichtung 33 und anschließend einem Sättiger 34 zur Sättigung mit Wasserdampf zugeführt. Der somit entschwefelte und gesättigte Erdgasstrom wird einem adiabaten Prereformer 35 zugeführt, in welchem höhere Kohlenwasserstoffe des Erdgasstroms 1a durch Dampfreformierung in ein Gemisch mit Methan, Wasserstoff und Kohlenstoffoxiden umgewandelt werden. Dieses Gemisch wird nun in einem Autothermreformer 36 in an sich bekannter Weise zu Synthesegas, im Wesentlichen bestehend aus Wasserstoff, Kohlenmonoxid und Kohlendioxid, umgewandelt. Bevorzugt ist daher, dass der Synthesegasstrom 2 in einer autothermen Reformierungsstufe 36a - welche hier durch den Autothermreformer 36 gebildet wird - aus dem Energieträgerstrom 1 gewonnen wird, wobei in der autothermen Reformierungsstufe 36a eine katalytische partielle Oxidation die für die endothermen Dampfreformierungsreaktionen erforderliche Wärme bereitstellt. Bevorzugt ist, dass der in der autothermen Reformierungsstufe 36a gewonnene Synthesegasstrom 2 ein molares Verhältnis, wiederum gegeben durch

$$S = \frac{n(H_2) - n(CO_2)}{n(CO) + n(CO_2)}$$ mit n in [mol], von S < 2 und insbesondere von S < 1,8 aufweist.

Im Anschluss an die autotherme Reformierungsstufe 36a wird der somit erhaltene Synthesegasstrom 2 in einer Abhitzeanordnung 37 gekühlt. Der Abhitzeanordnung prozesstechnisch nachgelagert kann eine weitere Kühlanordnung 38 zum Abkühlen des Synthesegasstroms 2 vorgesehen sein. Die Fig. 1 und 2 stellen als nachfolgende Prozessstufe den Synthesegaskompressor 24 aus der Fig. 3 bzw. 4 dar. Die Gesamtheit der Anordnungen zum Gewinnen des Synthesegasstroms 2 aus dem Energieträgerstrom 1 kann auch als Synthesegasquelle 2a bezeichnet werden. Diese umfasst bei den vorliegenden Ausführungsbeispielen die Entschwefelungsvorrichtung 33, den Sättiger 34, den Prereformer 35 sowie den Autothermreformer 36.

Regelmäßig wird bei der Herstellung des Synthesegases für den Synthesegasstrom 2 - und insbesondere bei der obigen autothermen Reformierung - nicht die für die Methanolsynthese optimale Stöchiometriezahl in dem Synthesegas erreicht. Daher kann es zweckmäßig sein, vor allem im Extremfall des Vorliegens von höheren Kohlenwasserstoffen, vor der Methanolsynthese weitere Maßnahmen zur entsprechenden Anpassung der Stöchiometriezahl vorzusehen. Insbesondere wenn das Synthesegas aus der Herstellung eine Stöchiometriezahl, welche hier durch die Formel

$$S = \frac{n(H_2) - n(CO_2)}{n(CO) + n(CO_2)}$$ - wobei n in [mol] gegeben ist - bestimmt wird, von kleiner als 2 aufweist, ist eine Erhöhung der Stöchiometriezahl des Synthesegasstroms auf größer 2 vorteilhaft.

Eine erste diesbezüglich bevorzugte Variante, welche in der Variante der Fig. 4 gezeigt ist, sieht dabei vor, dass aus dem Synthesegasstrom 2 vor Zuführung des wasserstoffhaltigen Stroms 3 ein Shift-Teilstrom 39 abgezweigt und einer Shiftvorrichtung 40 zugeführt wird, in welcher der Shift-Teilstrom 39 durch eine Wassergas-Shift Reaktion zumindest teilweise in Kohlenstoffdioxid und Wasserstoff umgewandelt wird, und dass das Kohlenstoffdioxid und der Wasserstoff aus der Shiftvorrichtung 40 der Wasserstoffrückgewinnungsstufe 4 zugeführt wird. Die Shiftvorrichtung 40 und die Wasserstoffrückgewinnungsstufe 4 wie bereits in den Fig. 1 und 2 dargestellt und oben beschrieben sind dabei in der Fig. 4 eingetragen. Das Kohlenstoffdioxid wird dann mit dem Abgas 11 entfernt, wohingegen der somit gewonnene Wasserstoff durch den wasserstoffhaltigen Strom 3 dem Synthesegasstrom 2 zugeführt wird und folglich für die Methanolsynthese zur Verfügung steht. Dieses Entfernen des Kohlenstoffdioxids und Beibehalten des Wasserstoffs führt zu einer Erhöhung der Stöchiometriezahl.

Alternativ oder zusätzlich kann die in der Fig. 3 dargestellte Variante vorgesehen sein, nach welcher aus dem Synthesegasstrom 2 ein Wasch-Teilstrom 41 ausgekreist wird und der Wasch-Teilstrom einer Waschvorrichtung 42 zum Auswaschen von Kohlenstoffdioxid aus dem Wasch-Teilstrom 41 zugeführt wird. Das Auswaschen von Kohlenstoffdioxid kann dabei auf im Prinzip beliebige Art und Weise geschehen. Auch diese Maßnahme erhöht die Stöchiometriezahl.

Die Speiseanordnung 2b kann nun die Synthesegasquelle 2a sowie die weiteren mit Bezug auf die Fig. 3 und 4 beschriebenen Vorrichtungen umfassen.

Die vorschlagsgemäße Anlage - für welche entsprechend je ein Ausführungsbeispiel in den Fig. 1 und 2 dargestellt ist - zur Synthese von Methanol umfasst eine Speiseanordnung 2b zum Bereitstellen eines Synthesegasstroms 2 mit Wasserstoff und Kohlenstoffoxiden. Die vorschlagsgemäße Anlage umfasst ebenso eine Wasserstoffrückgewinnungsstufe 4, aus der ein wasserstoffhaltiger Strom 3 dem Synthesegasstrom 2 zugeführt wird und umfasst ferner eine Hauptreaktorstufe 5, welcher der Synthesegasstrom 2 zugeführt wird und in welcher der Synthesegasstrom 2 katalytisch und teilweise in ein Gasgemisch 6 mit Wasser, Methanol und Restgas umgesetzt wird, wobei ein erster Teil 7 des Restgases der Wasserstoffrückgewinnungsstufe 4 zur Trennung in den wasserstoffhaltigen Strom 3 und in einen Abgasstrom 11 zugeführt wird.

Die vorschlagsgemäße Anlage ist dadurch gekennzeichnet, dass sie eine Nachreaktorstufe 8 aufweist, welcher ein zweiter Teil 10 des Restgases zur weiteren katalytischen und zumindest teilweisen Umsetzung in einen methanolhaltigen Produktstrom 9 zugeführt wird.

Weitere besondere und bevorzugte Ausgestaltungen und Merkmale der vorschlagsgemäßen Anlage ergeben sich aus den entsprechenden Ausgestaltungen und Merkmalen des vorschlagsgemäßen Verfahrens.

## Patentansprüche

1. Verfahren zur Synthese von Methanol, wobei einem Synthesegasstrom (2), welcher Wasserstoff und Kohlenstoffoxide umfasst, ein wasserstoffhaltiger Strom (3) aus einer Wasserstoffrückgewinnungsstufe (4) zugeführt wird und wobei der Synthesegasstrom (2) einer Hauptreaktorstufe (5) zur katalytischen und teilweisen Umsetzung des Synthesegasstroms (2) in ein Gasgemisch (6) mit Wasser, Methanol und Restgas zugeführt wird und wobei ein erster Teil (7) des Restgases der Wasserstoffrückgewinnungsstufe (4) zugeführt wird zur Trennung in den wasserstoffhaltigen Strom (3) und in einen Abgasstrom (11),
**dadurch gekennzeichnet,**
**dass** ein zweiter Teil (10) des Restgases einer Nachreaktorstufe (8) zur weiteren katalytischen und zumindest teilweisen Umsetzung in einen methanolhaltigen Produktstrom (9) zugeführt wird.

2. Verfahren nach Anspruch 1, **dadurch gekennzeichnet, dass** der Synthesegasstrom (2) vor Zuführung des wasserstoffhaltigen Stroms (3) ein molares Verhältnis, gegeben durch $S = \frac{n(H_2) - n(CO_2)}{n(CO) + n(CO_2)}$, von S < 2 aufweist und dass in der Haupt-reaktorstufe (5) ein molares Verhältnis von S > 2, vorzugsweise von S > 3, vorliegt.

3. Verfahren nach einem der Ansprüche 1 bis 2, **dadurch gekennzeichnet, dass** in der Nachreaktorstufe (8) ein molares Verhältnis S vorliegt, gegeben durch $S = \frac{n(H_2) - n(CO_2)}{n(CO) + n(CO_2)}$, welches größer ist, vorzugsweise mindestens um den Faktor 1,5 größer ist, als das in der Hauptreaktorstufe (5) vorliegende molare Verhältnis S, insbesondere, dass der molare Anteil von Kohlenstoffmonoxid an der Summe der Molmenge von Wasserstoff, Kohlenstoffmonoxid und Kohlenstoffdioxid in der Nachreaktorstufe (8) geringer ist als der molare Anteil von Kohlenstoffmonoxid an der Summe der Molmenge von Wasserstoff, Kohlenstoffmonoxid und Kohlenstoffdioxid in der Hauptreaktorstufe (5).

4. Verfahren nach einem der Ansprüche 1 bis 3, **da-**

**durch gekennzeichnet, dass** der erste Teil (7) des Restgases und der zweite Teil (10) des Restgases durch eine Stromaufteilung des Gasgemisches (6) in mindestens einen Hauptrecyclestrom (16), umfassend den zweiten Teil (10), zur Zuführung in die Nachreaktorstufe (8) und in einen Purgestrom (17), umfassend den ersten Teil (7), zur Zuführung zu der Wasserstoffrückgewinnungsstufe (4) getrennt werden, vorzugsweise, dass das Volumenverhältnis des Hauptrecyclestroms (16) zu dem Purgestrom (17) einstellbar ist, insbesondere, dass das Volumenverhältnis des Hauptrecyclestroms (16) zu dem Purgestrom (17) basierend auf einer Zusammensetzung des Synthesegasstroms (2) eingestellt wird.

5. Verfahren nach Anspruch 4, **dadurch gekennzeichnet, dass** der Purgestrom (17) vor Zuführung zu der Wasserstoffrückgewinnungsstufe (4) einer Waschstufe (18) zum Auswaschen von verbliebenem Methanol in dem Purgestrom (17) zugeführt wird

6. Verfahren nach Anspruch 4 oder 5, **dadurch gekennzeichnet, dass** der Hauptrecyclestrom (16) zur Komprimierung durch einen Recyclekompressor (19) gefahren wird und/oder dass der Hauptrecyclestrom (16) zur Kühlung des Gasgemischs (6) durch einen Hauptrecycle-Wärmetauscher (20) geführt wird.

7. Verfahren nach einem der Ansprüche 1 bis 6, **dadurch gekennzeichnet, dass** das Gasgemisch (6) gekühlt wird, insbesondere, dass das Gasgemisch (6) einer Trennstufe (12) zum Abtrennen eines Rohmethanolstroms (13) zugeführt wird, vorzugsweise, dass die Trennstufe (12) eine Abscheidestufe (14) aufweist, welche den Rohmethanolstrom (13) als Kondensat des Gasgemisches (6) abtrennt, weiter insbesondere, dass die Trennstufe (12) eine der Abscheidestufe (14) vorgelagerte Kühlstufe (15) zur Abkühlung des Gasgemisches (6), weiter vorzugsweise zur Abkühlung auf eine Kühltemperatur oberhalb des Siedepunkts von Methanol bei Normaldruck, aufweist.

8. Verfahren nach Anspruch 7, **dadurch gekennzeichnet, dass** die Kühlung des Gasgemischs (6) vor einer Aufteilung in den ersten Teil (7) und den zweiten Teil (10) des Restgases erfolgt, vorzugsweise, dass die Trennstufe (12) eine der Kühlstufe (15) prozesstechnisch nachgelagerte weitere Kühlstufe (21) aufweist, weiter vorzugsweise, dass die Kühlstufe (15) und die weitere Kühlstufe (21) das Gasgemisch (6) auf weniger als 45° C kühlen, insbesondere, dass nach Abtrennen des Rohmethanolstroms (13) das verbliebene Gasgemisch (6) in den Hauptrecyclestrom (16) und in den Purgestrom (17) getrennt wird.

9. Verfahren nach einem der Ansprüche 1 bis 8, **dadurch gekennzeichnet, dass** der Synthesegasstrom (2) zur Komprimierung durch einen Synthesegaskompressor (24) gefahren wird, vorzugsweise, dass der Synthesegasstrom (2) vor Zuführung zu der Hauptreaktorstufe (5) der Nachreaktorstufe (8) zu ihrer Gaskühlung zugeführt wird, insbesondere, dass der wasserstoffhaltige Strom (3) dem Synthesegasstrom (2) prozesstechnisch dem Synthesegaskompressor (24) vorgelagert zugeführt wird.

10. Verfahren nach einem der Ansprüche 1 bis 9, **dadurch gekennzeichnet, dass** der methanolhaltige Produktstrom (9) einer weiteren Trennstufe (25) zum Abtrennen eines weiteren Rohmethanolstroms (26) zugeführt wird, vorzugsweise, dass ein nach Abtrennen des weiteren Rohmethanolstroms (26) verbliebener Nebenrecyclestrom (27) dem Synthesegasstrom (2) zugeführt wird, vorzugsweise, dass der Nebenrecyclestrom (27) dem Synthesegasstrom (2) prozesstechnisch dem Synthesegaskompressor (24) nachgelagert zugeführt wird.

11. Verfahren nach Anspruch 10, **dadurch gekennzeichnet, dass** der Nebenrecyclestrom (27) einen Molanteil an Kohlenstoffdioxid aufweist, welcher mindestens doppelt so hoch wie der Molanteil des Nebenrecyclestroms (27) an Kohlenstoffmonoxid ist.

12. Verfahren nach einem der Ansprüche 1 bis 11, **dadurch gekennzeichnet, dass** der Synthesegasstrom (2) aus einem kohlenstoffhaltigen Energieträgerstrom (1) gewonnen wird, insbesondere, dass der kohlenstoffhaltige Energieträgerstrom (1) einen Erdgasstrom (1a) umfasst, vorzugsweise im Wesentlichen aus dem Erdgasstrom (1a) besteht, insbesondere, dass der Erdgasstrom (1a) aus einer Vielzahl von geografisch verteilten Erdgasvorkommen gespeist wird.

13. Verfahren nach Anspruch 12, **dadurch gekennzeichnet, dass** der Synthesegasstrom (2) in einer autothermen Reformierungsstufe (36a) aus dem Energieträgerstrom (1) gewonnen wird, wobei in der autothermen Reformierungsstufe (36a) eine katalytische partielle Oxidation die für die endothermen Dampfreformierungsreaktionen erforderliche Wärme bereitstellt, vorzugsweise, dass der in der autothermen Reformierungsstufe (36a) gewonnene Synthesegasstrom (2) ein molares Verhältnis, gegeben durch $S = \frac{n(H_2) - n(CO_2)}{n(CO) + n(CO_2)}$, von S < 2, insbesondere von S < 1,8, aufweist.

14. Verfahren nach einem der Ansprüche 1 bis 13, **dadurch gekennzeichnet, dass** aus dem Synthesegasstrom (2) ein Wasch-Teilstrom (41) ausgekreist wird und der Wasch-Teilstrom (41) einer Waschvorrichtung (42) zum Auswaschen von Kohlenstoffdioxid aus dem Wasch-Teilstrom (41) zugeführt wird.

15. Anlage zur Synthese von Methanol umfassend eine Speiseanordnung (2b) zum Bereitstellen eines Synthesegasstroms (2) mit Wasserstoff und Kohlenstoffoxiden, umfassend eine Wasserstoffrückgewinnungsstufe (4), aus der ein wasserstoffhaltiger Strom (3) dem Synthesegasstrom (2) zugeführt wird und umfassend eine Hauptreaktorstufe (5), welcher der Synthesegasstrom (2) zugeführt wird und in welcher der Synthesegasstrom (2) katalytisch und teilweise in ein Gasgemisch (6) mit Wasser, Methanol und Restgas umgesetzt wird, wobei ein erster Teil (7) des Restgases der Wasserstoffrückgewinnungsstufe (4) zur Trennung in den wasserstoffhaltigen Strom (3) und in einen Abgasstrom (11) zugeführt wird,
**dadurch gekennzeichnet,**
**dass** die Anlage eine Nachreaktorstufe (8) aufweist, welcher ein zweiter Teil (10) des Restgases zur weiteren katalytischen und zumindest teilweisen Umsetzung in einen methanolhaltigen Produktstrom (9) zugeführt wird.

## Claims

1. A method for the synthesis of methanol, wherein a hydrogen-containing stream (3) from a hydrogen recovery stage (4) is fed into a synthesis gas stream (2) which comprises hydrogen and carbon oxides, and wherein the synthesis gas stream (2) is fed to a primary reactor stage (5) for the catalytic and partial conversion of the synthesis gas stream (2) into a gas mixture (6) with water, methanol and residual gas, and wherein a first portion (7) of the residual gas is fed to the hydrogen recovery stage (4) for separation into the hydrogen-containing stream (3) and a waste gas stream (11),
**characterized in that**
a second portion (10) of the residual gas is fed to a secondary reactor stage (8) for further catalytic and at least partial conversion into a methanol-containing product stream (9).

2. The method according to claim 1, **characterized in that** the synthesis gas stream (2), prior to the feed of the hydrogen-containing stream (3), has a molar ratio, given by $S = \dfrac{n(H_2) - n(CO_2)}{n(CO) + n(CO_2)}$, of S < 2, and that a molar ratio of S > 2, preferably of S > 3, is provided in the primary reactor stage (5).

3. The method according to any one of the claims 1 to 2, **characterized in that** in the secondary reactor stage (8), a molar ratio S, given by $S = \dfrac{n(H_2) - n(CO_2)}{n(CO) + n(CO_2)}$, is provided which is greater, preferably greater by at least the factor 1.5, than the molar ratio S provided in the primary reactor stage (5), in particular, that the molar proportion of carbon monoxide in the sum of the molar amount of hydrogen, carbon monoxide and carbon monoxide in the secondary reactor stage (8) is smaller than the molar proportion of carbon monoxide in the sum of the molar amount of hydrogen, carbon monoxide and carbon monoxide in the primary reactor stage (5).

4. The method according to any one of the claims 1 to 3, **characterized in that** the first portion (7) of the residual gas and the second portion (10) of the residual gas are separated by means of a division of the stream of the gas mixture (6) into at least one primary recycling stream (16), comprising the second portion (10), for being fed to the secondary reactor stage (8), and a purge stream (17), comprising the first portion (7), for being fed to the hydrogen recovery stage (4), preferably, that the volume ratio of the primary recycling stream (16) to the purge stream (17) is adjustable, in particular, that the volume ratio of the primary recycling stream (16) to the purge stream (17) is adjusted based on a composition of the synthesis gas stream (2).

5. The method according to claim 4, **characterized in that** the purge stream (17), prior to being fed to the hydrogen recovery stage (4), is fed to a scrubbing stage (18) for scrubbing residual methanol from the purge stream (17).

6. The method according to claim 4 or 5, **characterized in that** the primary recycling stream (16) is moved through a recycling compressor (19) for compression, and/or that the primary recycling stream (16) is conducted through a primary recycling heat exchanger (20) for cooling the gas mixture (6).

7. The method according to any one of the claims 1 to 6, **characterized in that** the gas mixture (6) is cooled, in particular, that the gas mixture (6) is fed to a separation stage (12) for separating a crude-methanol stream (13), preferably, that the separation stage (12) has a deposition stage (14) which separates the crude-methanol stream (13) as a condensate of the gas mixture (6), more particularly, that the separation stage (12) has a cooling stage (15) upstream of the deposition stage (14) for cooling the gas mixture (6), more preferably for cooling it down to a cooling temperature above the boiling point of methanol at normal pressure.

**8.** The method according to claim 7, **characterized in that** the gas mixture (6) is cooled before being divided into the first portion (7) and the second portion (10) of the residual gas, preferably, that the separation stage (12) has a further cooling stage (21), which is downstream of the cooling stage (15) in terms of the process, more preferably, that the cooling stage (15) and the further cooling stage (21) cool the gas mixture (6) down to less than 45°C, in particular, that after the separation of the crude-methanol stream (13), the residual gas mixture (6) is divided into the primary recycling stream (16) and the purge stream (17).

**9.** The method according to any one of the claims 1 to 8, **characterized in that** the synthesis gas stream (2) is moved through a synthesis gas compressor (24) for compression, preferably, that the synthesis gas stream (2), prior to being fed to the primary reactor stage (5), is fed to the secondary reactor stage (8) for cooling it with gas, in particular, that the hydrogen-containing stream (3) is fed into the synthesis gas stream (2) upstream of the synthesis gas compressor (24) in terms of the process.

**10.** The method according to any one of the claims 1 to 9, **characterized in that** the methanol-containing product stream (9) is fed to another separation stage (25) for separating a further crude-methanol stream (26), preferably, that a secondary recycling stream (27) remaining after the separation of the further crude-methanol stream (26) is fed into the synthesis gas stream (2), preferably, that the secondary recycling stream (27) is fed into the synthesis gas stream (2) downstream of the synthesis gas compressor (24) in terms of the process.

**11.** The method according to claim 10, **characterized in that** the secondary recycling stream (27) has a molar proportion of carbon dioxide which is at least twice as large as the molar proportion of the secondary recycling stream (27) of carbon monoxide.

**12.** The method according to any one of the claims 1 to 11, **characterized in that** the synthesis gas stream (2) is obtained from a carbon-containing energy-carrier stream (1), in particular, that the carbon-containing energy-carrier stream (1) includes a natural-gas stream (1a), preferably substantially consists of the natural-gas stream (1a), in particular, that the natural-gas stream (1a) is supplied from a plurality of geographically distributed natural gas deposits.

**13.** The method according to claim 12, **characterized in that** the synthesis gas stream (2) is obtained from the energy-carrier stream (1) in an autothermic reformation stage (36a), wherein in the autothermic reformation stage (36a), a catalytic partial oxidation provides the heat required for the endothermic steam reformation reactions, preferably, that the synthesis gas stream (2) obtained in the autothermic reformation stage (36a) has a molar ratio, given by

$$S = \frac{n(H_2) - n(CO_2)}{n(CO) + n(CO_2)},$$

of S < 2, in particular of S < 1.8.

**14.** The method according to any one of the claims 1 to 13, **characterized in that** a partial scrubbing stream (41) is removed from the synthesis gas stream (2) and the partial scrubbing stream (41) is fed to a scrubbing device (42) for scrubbing carbon dioxide from the partial scrubbing stream (41).

**15.** A system for the synthesis of methanol, comprising a feeding assembly (2b) for providing a synthesis gas stream (2) with hydrogen and carbon oxides, comprising a hydrogen recovery stage (4) from which a hydrogen-containing stream (3) is fed into the synthesis gas stream (2), and comprising a primary reactor stage (5) to which the synthesis gas stream (2) is fed and in which the synthesis gas stream (2) is catalytically and partially converted into a gas mixture (6) with water, methanol and residual gas, wherein a first portion (7) of the residual gas is fed to the hydrogen recovery stage (4) for separation into the hydrogen-containing stream (3) and a waste gas stream (11),
**characterized in that**
the system has a secondary reactor stage (8) to which a second portion (10) of the residual gas is fed for further catalytic and at least partial conversion into a methanol-containing product stream (9).

**Revendications**

**1.** Procédé pour la synthèse de méthanol, dans lequel un flux de gaz de synthèse (2) qui comprend de l'hydrogène et des oxydes de carbone reçoit un flux (3) contenant de l'hydrogène et provenant d'un étage de récupération d'hydrogène (4), et dans lequel le flux de gaz de synthèse (2) est amené à un étage de réacteur principal (5) pour la transformation catalytique et partielle du flux de gaz de synthèse (2) en un mélange gazeux (6) présentant de l'eau, du méthanol et du gaz résiduel, et dans lequel une première partie (7) du gaz résiduel est amenée à l'étage de récupération d'hydrogène (4) pour la séparation en ledit flux contenant de l'hydrogène (3) et en un flux de gaz d'échappement (11),
**caractérisé par le fait**
**qu'**une deuxième partie (10) du gaz résiduel est amenée à un étage de post-réacteur (8) pour l'autre transformation catalytique et au moins partielle en un flux de produit contenant du méthanol (9).

**2.** Procédé selon la revendication 1, **caractérisé par le fait que**, avant d'amener le flux contenant de l'hydrogène (3), le flux de gaz de synthèse (2) présente un rapport molaire, donné par $S = \frac{n(H_2)-n(CO_2)}{n(CO)+n(CO_2)}$, de S < 2, et que, dans ledit étage de réacteur principal (5), il y a un rapport molaire de S > 2, de préférence de S > 3.

**3.** Procédé selon l'une quelconque des revendications 1 à 2, **caractérisé par le fait que**, dans ledit étage de post-réacteur (8), il y a un rapport molaire de S, donné par $S = \frac{n(H_2)-n(CO_2)}{n(CO)+n(CO_2)}$, qui est supérieur, de préférence supérieur d'au moins le facteur 1,5, au rapport molaire S présent dans l'étage de réacteur principal (5), en particulier que la proportion molaire de monoxyde de carbone à la somme de la quantité molaire d'hydrogène, de monoxyde de carbone et de dioxyde de carbone dans l'étage de post-réacteur (8) est inférieure à la proportion molaire de monoxyde de carbone à la somme de la quantité molaire d'hydrogène, de monoxyde de carbone et de dioxyde de carbone dans l'étage de réacteur principal (5).

**4.** Procédé selon l'une quelconque des revendications 1 à 3, **caractérisé par le fait que** la première partie (7) du gaz résiduel et la deuxième partie (10) du gaz résiduel sont séparées, par une distribution de flux du mélange gazeux (6), en au moins un flux de recyclage principal (16), comprenant la deuxième partie (10), pour être amené à l'étage de post-réacteur (8), et en un flux de purge (17), comprenant la première partie (7), pour être amené à l'étage de récupération d'hydrogène (4), de préférence que le rapport volumique du flux de recyclage principal (16) au flux de purge (17) est réglable, en particulier que le rapport volumique du flux de recyclage principal (16) au flux de purge (17) est réglé sur la base d'une composition du flux de gaz de synthèse (2).

**5.** Procédé selon la revendication 4, **caractérisé par le fait que**, avant d'être amené à l'étage de récupération d'hydrogène (4), le flux de purge (17) est amené à un étage de lavage (18) pour enlever par lavage du méthanol résiduel dans le courant de purge (17).

**6.** Procédé selon la revendication 4 ou 5, **caractérisé par le fait que**, pour le comprimer, on fait passer le flux de recyclage principal (16) à travers un compresseur de recyclage (19) et/ou que, pour refroidir le mélange de gaz (6), on fait passer le flux de recyclage principal (16) à travers un échangeur de chaleur de recyclage principal (20).

**7.** Procédé selon l'une quelconque des revendications 1 à 6, **caractérisé par le fait que** le mélange gazeux (6) est refroidi, en particulier que le mélange gazeux (6) est amené à un étage de séparation (12) pour séparer un flux de méthanol brut (13), de préférence que ledit étage de séparation (12) comprend un étage d'isolation (14) qui sépare le flux de méthanol brut (13) en tant que produit de condensation du mélange gazeux (6), en outre en particulier que l'étage de séparation (12) comprend un étage de refroidissement (15) situé en amont de l'étage d'isolation (14), pour le refroidissement du mélange gazeux (6), en outre de préférence pour le refroidissement à une température de refroidissement au-dessus du point d'ébullition de méthanol à la pression atmosphérique.

**8.** Procédé selon la revendication 7, **caractérisé par le fait que** le refroidissement du mélange gazeux (6) se fait avant la séparation en ladite première partie (7) et ladite deuxième partie (10) du gaz résiduel, de préférence que l'étage de séparation (12) comprend un autre étage de refroidissement (21) qui, quant à la technique du processus, est situé en aval de l'étage de refroidissement (15), en outre de préférence que l'étage de refroidissement (15) et l'autre étage de refroidissement (21) refroidissent le mélange gazeux (6) à moins de 45 °C, en particulier que, après la séparation du flux de méthanol brut (13), le reste du mélange de gaz (6) est divisé en ledit flux de recyclage principal (16) et en ledit flux de purge (17).

**9.** Procédé selon l'une quelconque des revendications 1 à 8, **caractérisé par le fait que**, pour le comprimer, on fait passer le flux de gaz de synthèse (2) à travers un compresseur de gaz de synthèse (24), de préférence que le flux de gaz de synthèse (2), avant d'être amené à l'étage de réacteur principal (5), est amené à l'étage de post-réacteur (8) pour son refroidissement de gaz, en particulier que le flux contenant de l'hydrogène (3) est amené au flux de gaz de synthèse (2) en amont du compresseur de gaz de synthèse (24) quant à la technique de processus.

**10.** Procédé selon l'une quelconque des revendications 1 à 9, **caractérisé par le fait que** le flux de produit contenant du méthanol (9) est amené à un autre étage de séparation (25) pour séparer un autre flux de méthanol brut (26), de préférence qu'un flux de recyclage secondaire (27) qui reste après avoir séparé ledit autre flux de méthanol brut (26) est amené au flux de gaz de synthèse (2), de préférence que ledit flux de recyclage secondaire (27) est amené au flux de gaz de synthèse (2) en aval du compresseur de gaz de synthèse (24) quant à la technique de processus.

**11.** Procédé selon la revendication 10, **caractérisé par le fait que** le flux de recyclage secondaire (27) présente une proportion molaire en dioxyde de carbone qui est au moins deux fois supérieure à la proportion molaire en monoxyde de carbone du flux de recyclage secondaire (27).

**12.** Procédé selon l'une quelconque des revendications 1 à 11, **caractérisé par le fait que** le flux de gaz de synthèse (2) est obtenu à partir d'un flux de vecteur d'énergie (1) contenant du carbone, en particulier que le flux de vecteur d'énergie (1) contenant du carbone comprend un flux de gaz naturel (1a), de préférence constitué pour l'essentiel par le flux de gaz naturel (1a), en particulier que le flux de gaz naturel (1a) est alimenté à partir d'une pluralité de gisements de gaz naturel distribués géographiquement.

**13.** Procédé selon la revendication 12, **caractérisé par le fait que** le flux de gaz de synthèse (2) est obtenu dans un étage de reformage auto-thermique (36a) à partir du flux de vecteur d'énergie (1), dans lequel, dans ledit étage de reformage auto-thermique (36a), une oxydation catalytique partielle fournit la chaleur nécessaire aux réactions de vaporeformage endothermiques, de préférence que le flux de gaz de synthèse (2) obtenu dans l'étage de reformage auto-thermique (36a) présente un rapport molaire, donné par $S = \frac{n(H_2) - n(CO_2)}{n(CO) + n(CO_2)}$, de S < 2, en particulier de S < 1,8.

**14.** Procédé selon l'une quelconque des revendications 1 à 13, **caractérisé par le fait qu'**un flux partiel de lavage (41) est séparé à partir du flux de gaz de synthèse (2), et que le flux partiel de lavage (41) est amené à un dispositif de lavage (42) pour enlever par lavage, dudit flux partiel de lavage (41), du dioxyde de carbone .

**15.** Installation pour la synthèse de méthanol, comprenant un agencement d'alimentation (2b) pour fournir un flux de gaz de synthèse (2) comprenant de l'hydrogène et des dioxydes de carbone, comprenant un étage de récupération d'hydrogène (4) à partir duquel un flux contenant de l'hydrogène (3) est amené au flux de gaz de synthèse (2), et comprenant un étage de réacteur principal (5) auquel est amené le flux de gaz de synthèse (2) et dans lequel le flux de gaz de synthèse (2) est transformé de façon catalytique et partielle en un mélange gazeux (6) présentant de l'eau, du méthanol et du gaz résiduel, dans laquelle une première partie (7) du gaz résiduel est amenée à l'étage de récupération d'hydrogène (4) pour la séparation en ledit flux contenant de l'hydrogène (3) et en un flux de gaz d'échappement (11), **caractérisée par le fait que** l'installation présente un étage de post-réacteur (8) auquel est amenée une deuxième partie (10) du gaz résiduel pour l'autre transformation catalytique et au moins partielle en un flux de produit contenant du méthanol (9).

Fig. 1

Fig. 2

# Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 9621634 A
- WO 2006126017 A1